Europäisches Patentamt

European Patent Office

Office européen des brevets

(19)

(11) Veröffentlichungsnummer : **0 417 680 B1**

## (12) EUROPÄISCHE PATENTSCHRIFT

(45) Veröffentlichungstag der Patentschrift :
**10.03.93 Patentblatt 93/10**

(51) Int. Cl.⁵ : **C07C 17/20, C07C 19/08, B01J 23/26**

(21) Anmeldenummer : **90117355.9**

(22) Anmeldetag : **10.09.90**

(54) **Verfahren zur Herstellung von 1,1,1,2-Tetrafluorethan.**

(30) Priorität : **13.09.89 DE 3930507**

(43) Veröffentlichungstag der Anmeldung :
**20.03.91 Patentblatt 91/12**

(45) Bekanntmachung des Hinweises auf die
Patenterteilung :
**10.03.93 Patentblatt 93/10**

(84) Benannte Vertragsstaaten :
**BE DE DK ES FR GB GR IT NL**

(56) Entgegenhaltungen :
**EP-A- 0 130 532**
**EP-A- 0 317 981**
**FR-A- 2 381 006**
**US-A- 3 992 325**

(56) Entgegenhaltungen :
**PATENT ABSTRACTS OF JAPAN, Band 7, Nr. 46 (C-153)(1191), 23. Februar 1983; & JP-A-57197232**
**PATENT ABSTRACTS OF JAPAN, Band 4, Nr. 54 (C-8)(536), 23. April 1980; & JP-A-5527139**

(73) Patentinhaber : **HOECHST AKTIENGESELLSCHAFT**
**Postfach 80 03 20**
**W-6230 Frankfurt am Main 80 (DE)**

(72) Erfinder : **Wanzke, Wolfgang, Dr.**
**Johannesallee 12**
**W-6230 Frankfurt am Main (DE)**
Erfinder : **Siegemund, Günter, Dr.**
**Frankfurter Strasse 21**
**W-6238 Hofheim am Taunus (DE)**
Erfinder : **Schmieder, Wilfried, Dr.**
**Zeilsheimer Strasse 43**
**W-6238 Hofheim am Taunus (DE)**

EP 0 417 680 B1

## Beschreibung

Die Erfindung betrifft ein Verfahren zur Herstellung von 1,1,1,2-Tetrafluorethan durch Umsetzung von 1,1,1-Trifluor-2-chlorethan mit Fluorwasserstoff in der Gasphase.

1,1,1,2-Tetrafluorethan (das im folgenden auch als Tetrafluorethan bezeichnet wird) kann als chlorfreier Ersatzstoff für Difluordichlormethan (R 12) in der Kälte- und Klimatechnik eingesetzt werden.

Es ist bereits bekannt, daß Tetrafluorethan bei der Umsetzung von 1,1,1-Trifluor-2-chlorethan (im folgenden auch als Trifluorchlorethan bezeichnet) mit Fluorwasserstoff an geeigneten Katalysatoren sowohl in der Gasphase als auch in flüssiger Phase erhalten werden kann. Als Katalysatoren für die Gasphasenreaktion sind vorwiegend Feststoffe beschrieben worden. die entweder ganz aus Chrom(III)verbindungen bestehen oder ein Chrom(III)salz auf einem Trägermaterial wie Aluminiumoxid enthalten. Die DE-OS 2 806 865 (= US-PS 4 129 603) beschreibt die Verwendung eines Chromoxid-Katalysators, der bevorzugt durch Behandlung einer Chromhydroxidpaste mit Dampf erhalten und anschließend mit Fluorwasserstoff in ein Chromoxifluorid umgewandelt wird. In der DE-PS 2 932 934 wird die Verwendung verschiedener Chrom(III)verbindungen als Katalysatoren beschrieben, z.B. Chrom(III)fluorid oder andere Salze, die durch eine Fluorwasserstoff-Behandlung in Chromoxifluorid oder Chrom(III)fluorid umgewandelt werden.

Die genannten Verfahren weisen noch verschiedene Nachteile auf, die vor allem für eine technische Herstellung von Tetrafluorethan ungünstig sind. Neben Tetrafluorethan entstehen nämlich noch andere Verbindungen, unter anderem 1,1-Difluor-2-chlorethen, das vom gewünschten Produkt destillativ schwierig abzutrennen ist.

Nach dem Verfahren der DE-OS 2 806 865 wird entweder ein zweiter Reaktor benötigt, um das im Rohprodukt enthaltene 1,1-Difluor-2-chlorethen bei niedrigeren Temperaturen wieder umzusetzen, oder es muß naßchemisch durch eine Permanganat-Lösung abgetrennt werden, was in beiden Fällen einen erheblichen zusätzlichen Aufwand erfordert. Die Selektivitäten, die mit dem Chromoxifluorid-Katalysator vor der Abtrennung von 1,1-Difluor-2-chlorethen erzielt werden, liegen bei 91-95 %.

Die in der DE-PS 2 932 934 beschriebenen Katalystoren erreichen bei Temperaturen um 400 °C kurzfristig eine höhere Selektivität für Tetrafluorethan (bis 98 %), wobei der Restgehalt an 1,1-Difluor-2-chlorethen nicht angegeben wird. Die verwendeten Chrom(III)-Katalysatoren verlieren jedoch bereits innerhalb von 44 Stunden merklich an Aktivität, was durch die kontinuierliche Dosierung einer bestimmten Menge an Sauerstoff kompensiert werden kann. Die zusätzliche Dosierung von Sauerstoff in den Reaktor bringt jedoch neben dem erhöhten technischen Aufwand noch weitere Probleme bei der Aufarbeitung des Produkts mit sich. Sauerstoff erschwert die Kompression oder Kondensation des Produkts und ist in gelöster Form bei der Anwendung von Tetrafluorethan in der Kälte- und Klimatechnik sehr nachteilig. Bei einer Überdosierung von Sauerstoff in den Reaktor wird außerdem die Selektivität für Tetrafluorethan wieder geringer.

Der von Marangoni et. al. (Chim. Ind. 64, 135 (1982)) beschriebene Katalysator aus gefälltem Chromhydroxid erreicht bei 350 °C über 60 Stunden nur eine Tetrafluorethan-Selektivität von 79 %.

Es wurde nun gefunden, daß der in der EP-PS 130 532 (= US-PS 4 547 483) beschriebene Katalysator, bestehend aus Magnesiumfluorid und einer fluorhaltigen Chrom(III)verbindung, die Umwardlung von Trifluorchlorethan in Tetrafluorethan überraschend selektiv und mit länger anthaltender Aktivität ermöglicht. In der noch nicht veröffentlichten deutschen Patentanmeldung P 39 23 256.5 wird dieser Katalysator zur Herstellung des Ausgangsmaterials 1,1,1-Trifluor-2-chlorethan eingesetzt.

Gegenstand der Erfindung ist ein Verfahren zur Herstellung von 1,1,1,2-Tetrafluorethan aus 1,1,1-Trifluor-2-chlorethan und Fluorwasserstoff un der Gasphase, dadurch gekennzeichnet, daß ein Chrom und Magnesium enthaltender Katalysator verwendet wird, der dadurch erhältlich ist, daß man Chrom(III)hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500 °C mit Fluorwasserstoff behandelt.

Der Katalysator für die erfindungsgemäße Gasphasenreaktion ist unter erhöhtem Druck besonders effektiv, da er den Gehalt an olefinischen Nebenprodukten im Produktgemisch, insbesondere an 1,1-Difluor-2-chlorethan, stark vermindert. Auf diese Weise kann der Olefingehalt im Rohprodukt auf Werte unter 100 ppm gesenkt werden, ohne daß ein zweiter Reaktor mit niedrigerer Temperatur oder eine chemische Wäsche eingesetzt wird, wie dies in der DE-OS 2 806 865 beschrieben ist. Während der Reaktion wird im Reaktor durch ein Regelventil ein Druck von 1-26 bar eingestellt, bevorzugt 5-15 bar.

Bei der erfindungsgemäßen Gasphasenreaktion zeigt der Katalysator gegenüber dem in der DE-PS 2 932 934 beschriebenen Katalysator über einen längeren Zeitraum keinen Aktivitätsverlust, so daß er ohne die zusätzliche Dosierung von Sauerstoff auskommt und somit die genannten technischen Nachteile entfallen.

Das erfindungsgemäße Verfahren kann beispielsweise so durchgeführt werden, daß die Ausgangsstoffe Trifluorchlorethan und Fluorwasserstoff kontinuierlich in einen Verdampfer aus Edelstahl oder Nickel gefördert werden. Die Temperatur des Verdampfers ist nicht kritisch, muß jedoch ausreichen, um beide Komponenten bei dem gewählten Durck vollständig in die Gasphase zu überführen. Die gasförmigen Ausgangsstoffe gelangen, gegebenenfalls über eine Vorheizstrecke und einen Gasmischer, in den Reaktor, der eine Schüttung des in der EP-OS 130 532 beschriebenen Katalysators enthält. Der Reaktor besteht ebenfalls aus Edelstahl oder Nickel und kann in verschiedenen technischen Ausführungen eingesetzt werden, z.B. als Schacht-, Röhren- oder Ringspaltreaktor.

Die Temperatur der Katalystorschüttung wird durch Beheizen des Reaktors bei 250-450 °C gehalten, bevorzugt bei 320-380 °C.

Um bei den angegebenen Reaktionstemperaturen einen möglichst hohen Umsatz an Trifluorchlorethan und eine sehr hohe Selektivität für Tetrafluorethan zu erzielen, wird der Fluorwasserstoff bevorzugt im Überschuß eingesetzt. Das Molverhältnis Fluorwasserstoff/Trifluorchlorethan ist i.allg. mindestens 1:1; nach oben ist das Molverhältnis nur durch wirtschaftliche Überlegungen begrenzt. Vorzugsweise liegt es bei 2:1 bis 10:1, insbesondere 4:1 bis 8:1.

Nicht umgesetztes Trifluorchlorethan kann wieder in den Reaktor zurückgeführt werden.

Das erfindungsgemäße Verfahren wird durch die nachfolgenden Beispiele näher erläutert.

**Versuchsbericht**

**A (Katalysatorherstellung gemäß EP-PS 130 532)**

200 g $(Cr(NO_3)_3$ x 9 $H_2O$ wurden in 1 l Wasser gelöst. Diese Lösung wurde zu einer Mischung von 500 g Magnesiumoxid und 240 g Graphit gegeben und die dabei entstehende pastöse Masse innig verknetet.

Anschließend wurde das pastöse Reaktionsprodukt zu Würfelförmlingen (0,5 cm Kantenlänge) granuliert und 16 Stunden bei 100 °C getrocknet.

1 l (Schüttvolumen) der getrockneten Katalysatorkörper (= 600 g) wurden in einem Rohr aus Nickel oder VA-Stahl mit 5 cm lichter Weite und 100 cm Länge bei 200 °C mit 15 mol Fluorwasserstoff behandelt. Die Dauer der Fluorwasserstoffbehandlung betrug ca. 6 Stunden. Dabei wurde der Fluorwasserstoff mit $N_2$ verdünnt. Der erhaltene Fluorierungskatalysator wies einen Chromgehalt von 2,3 Gew.-% auf.

**B (Katalysatorherstellung gemäß EP-PS 130 532)**

200 g $Cr(NO_3)_3$ x 9 $H_2O$ wurden in 278 ml Wasser aufgelöst. Diese Lösung wurde zu einer Mischung von 138 g Magnesiumoxid und 136 g Graphit gegeben. Die Weiterverarbeitung und Fluorwasserstoffbehandlung erfolgte gemäß Versuchsbericht A. Der fertige Fluorierungskatalysator enthielt 4,3 Gew.-% Chrom.

**Beispiel 1**

120 g Trifluorchlorethan und 120 g Fluorwasserstoff (Molverhältnis 1 : 5,9) wurden pro Stunde in gasförmigem Zustand über 1 l des nach Versuchsbericht A hergestellten Katalysators in einem Rohrreaktor geleitet, der durch eine elektrische Heizwicklung auf einer Temperatur von 360 °C gehalten wurde.

Der Rohrreaktor war der gleiche, der schon zur Fluorwasserstoffbehandlung bei der Herstellung des Katalysators eingesetzt wurde.

Die den Reaktor verlassenden gasförmigen Reaktionsprodukte wurden einer mit Wasser oder Kalilauge gefüllten Waschvorlage zugeführt, in der entstandener Chlorwasserstoff und überschüssiger Fluorwasserstoff aufgefangen wurden.

Die gasförmigen, nicht wasserlöslichen Reaktionsprodukte wurden gaschromatographisch analysiert.

Nach 6 Stunden bei Normaldruck betrug der Umsatz 27,4 %, bezogen auf eingesetztes Trifluorchlorethan. Die Selektivität für Tetrafluorethan war 96,4 %, bezogen auf umgesetztes Trifluorchlorethan.

Das Reaktionsprodukt enthielt 26,4 Gew.-% $CF_3$-$CH_2F$, 0,23 Gew.-% $CF_2$=CHCl und 0,77 Gew.-% andere Komponenten neben nicht umgesetztem $CF_3$-$CH_2Cl$.

**Beispiel 2**

Der gemäß Versuchsbericht A hergestellte Katalysator wurde in derselben Versuchsanordnung wie in Beispiel 1 zur Umsetzung von Trifluorchlorethan mit Fluorwasserstoff eingesetzt. Es wurden 120 g pro Stunde Trifluorchlorethan und 120 g pro Stunde Fluorwasserstoff gasförmig bei 360 °C und 10 bar Druck über den Katalysator geleitet.

Nach 20 Stunden betrug der Umsatz 26,6 %, bezogen auf eingesetztes Trifluorchlorethan. Die Selektivität für Tetrafluorethan war 97,5 %, bezogen auf umgesetztes Trifluorchlorethan.

Das Reaktionsprodukt enthielt 25,9 Gew.-% $CF_3$-$CH_2F$, 0,009 Gew.-% $CF_2$=CHCl und 0,69 Gew.-% andere Komponenten neben nicht umgesetztem $CF_3$-$CH_2Cl$.

**Beispiel 3**

Der gemäß Versuchsbericht A hergestellte Katalysator wurde in derselben Versuchsanordnung wie in Beispiel 1 zur Umsetzung von Trifluorchlorethan mit Fluorwasserstoff eingesetzt. Es wurden 120 g pro Stunde Trifluorchlorethan und 160 g pro Stunde Fluorwasserstoff (Molverhältnis 1 : 7,9) gasförmig bei 360 °C und 10 bar Druck über den Katalysator geleitet.

Nach 6 Stunden betrug der Umsatz 28,3 %, bezogen auf eingesetztes Trifluorchlorethan. Die Selektivität für Tetrafluorethan war 97,9 %, bezogen auf umgesetztes Trifluorchlorethan.

Das Reaktionsprodukt enthielt 27,7 Gew.-% $CF_3$-$CH_2F$, 0,006 Gew.-% $CF_2$=CHCl und 0,6 Gew.-% andere Komponenten neben nicht umgesetztem $CF_3$-$CH_2Cl$.

**Beispiel 4**

Der gemäß Versuchsbericht A hergestellte Katalysator wurde in derselben Versuchsanordnung wie in Beispiel 1 zur Umsetzung von Trifluorchlorethan mit Fluorwasserstoff eingesetzt. Es wurden 120 g pro Stunde Trifluorchlorethan und 120 g pro Stunde Fluorwasserstoff gasförmig bei 360 °C und 10 bar Druck über den Katalysator geleitet. Tabelle 1 zeigt den Umsatz an Trifluorchlorethan und die Selektivität für Tetrafluorethan in Abhängigkeit von der Reaktionszeit.

## Tabelle 1

| Reaktionszeit (h) | Umsatz (%) | Selektivität (%) |
|---|---|---|
| 5 | 27,1 | 97,2 |
| 22 | 26,5 | 96,7 |
| 32 | 26,1 | 97,1 |
| 55 | 26,4 | 97,5 |
| 81 | 26,6 | 97,2 |
| 90 | 26,5 | 97,3 |

**Beispiel 5**

Der gemäß Versuchsbericht B hergestellte Katalysator wurde in derselben Versuchsanordung wie in Beispiel 1 zur Umsetzung von Trifluorchlorethan mit Fluorwasserstoff eingesetzt. Es wurden 120 g pro Stunde Trifluorchlorethan und 120 g pro Stunde Fluorwasserstoff gasförmig bei 360 °C über den Katalysator geleitet.

Nach 5 Stunden betrug der Umsatz 29,2 %, bezogen auf eingesetztes Trifluorchlorethan. Die Selektivität für Tetrachlorethan war 95,7 %, bezogen auf umgesetztes Trifluorchlorethan.

Das Reaktionsprodukt enthielt 27,9 Gew.-% $CF_3$-$CH_2F$, 0,28 Gew.-% $CF_2$=CHCl und 1,02 Gew.-% andere Komponenten neben nicht umgesetztem $CF_3$-$CH_2Cl$.

**Beispiel 6**

Der gemäß Versuchsbericht A hergestellte Katalysator wurde in derselben Versuchsanordnung wie in Beispiel 1 zur Umsetzung von Trifluorchlorethan mit Fluorwasserstoff eingesetzt. Es wurden 120 g pro Stunde Trifluorchlorethan und 120 g pro Stunde Fluorwasserstoff gasförmig bei 420 °C über den Katalysator geleitet.

Nach 5 Stunden betrug der Umsatz 36,3 %, bezogen auf eingesetztes Trifluorchlorethan. Die Selektivität für Tetrafluorethan war 81,5 %, bezogen auf umgesetztes Trifluorchlorethan.

Das Reaktionsprodukt enthielt 29,6 Gew.-% $CF_3$-$CH_2F$, 2,2 Gew.-% $CF_2$=CHCl und 4,5 Gew-% andere Komponenten neben nicht umgesetztem $CF_3$-$CH_2Cl$.

## Patentansprüche

1. Verfahren zur Herstellung von 1,1,1,2-Tetrafluorethan aus 1,1,1-Trifluor-2-chlorethan und Fluorwasserstoff in der Gasphase, dadurch gekennzeichnet, daß ein Chrom und Magnesium enthaltender Katalysator verwendet wird, der dadurch erhältlich ist, daß man Chrom(III)-Hydroxid ausfällt, indem man 1 Mol eines wasserlöslichen Chrom(III)-Salzes mit mindestens 1,5 Mol Magnesiumhydroxid oder Magnesiumoxid in Gegenwart von Wasser umsetzt, die Reaktionsmischung in einen Teig überführt, der Chromhydroxid und ein Magnesiumsalz enthält, und dann den Teig trocknet und bei Temperaturen von 20 bis 500 °C mit Fluorwasserstoff behandelt.

2. Verfahren nach Anspruch 1, dadurch gekennzeichnet, daß die Reaktion von 1,1,1-Trifluor-2-chlorethan mit Fluorwasserstoff im Temperaturbereich von 250-450 °C durchgeführt wird.

3. Verfahren nach Anspruch 1, dadurch gekennzeichnet, daß die Reaktion von 1,1,1-Trifluor-2-chlorethan mit Fluorwasserstoff im Temperaturbereich von 320-380 °C durchgeführt wird.

4. Verfahren nach einem der Ansprüche 1 bis 3, dadurch gekennzeichnet, daß die Reaktion von 1,1,1-Trifluor-2-chlorethan mit Fluorwasserstoff unter einem Druck von 1-26 bar durchgeführt wird.

5. Verfahren nach einem der Ansprüche 1 bis 3, dadurch gekennzeichnet, daß die Reaktion von 1,1,1-Trifluor-2-chlorethan mit Fluorwasserstoff unter einem Druck von 5-15 bar durchgeführt wird.

6. Verfahren nach einem der Ansprüche 1 bis 5, dadurch gekennzeichnet, daß Fluorwasserstoff und 1,1,1-Trifluor-2-chlorethan in einem Molverhältnis von 4 : 1 bis 8 : 1 eingesetzt werden.

## Claims

1. A process for the preparation of 1,1,1,2-tetrafluoroethane from 1,1,1-trifluoro-2-chloroethane and hydrogen fluoride in the gas phase, which process comprises using a catalyst containing chromium and magnesium which is obtainable by precipitating chromium(III) hydroxide by reacting 1 mol of a water-soluble chromium(III) salt with at least 1.5 mol of magnesium hydroxide or magnesium oxide in the presence of water, making the reaction mixture into a paste containing chromium hydroxide and a magnesium salt, and then drying the paste and treating it at temperatures of 20 to 500°C with hydrogen fluoride.

2. The process as claimed in claim 1, wherein the reaction of 1,1,1-trifluoro-2-chloroethane with hydrogen fluoride is carried out in the temperature region of 250-450°C.

3. The process as claimed in claim 1, wherein the reaction of 1,1,1-trifluoro-2-chloroethane with hydrogen fluoride is carried out in the temperature region of 320-380°C.

4. The process as claimed in one of claims 1 to 3, wherein the reaction of 1,1,1-trifluoro-2-chloroethane with hydrogen fluoride is carried out under a pressure of 1-26 bar.

5. The process as claimed in one of claims 1 to 3, wherein the reaction of 1,1,1-trifluoro-2-chloroethane with hydrogen fluoride is carried out under a pressure of 5-15 bar.

6. The process as claimed in one of claims 1 to 5, wherein hydrogen fluoride and 1,1,1-trifluoro-2-chloroethane are used in a molar ratio of 4:1 to 8:1.

## Revendications

1. Procédé pour fabriquer du 1,1,1,2-tétrafluoréthane à partir de 1,1,1-trifluoro-2-chloréthane et de fluorure d'hydrogène en phase gazeuse , caractérisé en ce qu'on utilise un catalyseur contenant du chrome et du magnésium, que l'on peut obtenir en précipitant de l'hydroxyde de chrome-(III), en faisant réagir une mole d'un sel hydrosoluble de chrome-(III) avec au moins 1,5 mole d'hydroxyde de magnésium ou d'oxyde de magnésium en présence d'eau, en transformant le mélange réactionnel en une pâte, qui contient de l'hydroxyde de chrome et un sel de magnésium, puis en séchant la pâte et en la traitant par du fluorure d'hydrogène à des températures de 20 à 500°C.

2. Procédé selon la revendication 1, caractérisé en ce qu'on effectue la réaction du 1,1,1-trifluoro-2-chloréthane  avec le fluorure d'hydrogène dans un domaine de la température compris entre 250 et 450°C.

3. Procédé selon la revendication 1, caractérisé en ce qu'on effectue la réaction du 1,1,1-trifluoro-2-chloréthane  avec le fluorure d'hydrogène dans un domaine de la température allant de 320 à 380°C.

4. Procédé selon l'une des revendications 1 à 3, caractérisé en ce qu'on effectue la réaction du 1,1,1-trifluoro-2-chloréthane avec le fluorure d'hydrogène sous une pression de 1 à 26 bars.

5. Procédé selon l'une des revendications 1 à 3, caractérisé en ce qu'on effectue la réaction du 1,1,1-trifluoro-2-chloréthane avec le fluorure d'hydrogène sous une pression de 5 à 15 bars.

6. Procédé selon l'une des revendications 1 à 5, caractérisé en ce qu'on utilise le fluorure d'hydrogène et le 1,1,1-trifluoro-2-chloréthane selon un rapport molaire de 4:1 à 8:1.